# EUROPÄISCHE PATENTSCHRIFT

(11) **EP 1 556 126 B1**
(45) Veröffentlichungstag und Bekanntmachung des Hinweises auf die Patenterteilung: **16.08.2006**
(21) Anmeldenummer: 03772271.7
(22) Anmeldetag: 29.10.2003
(51) Int. Cl.: A61M 39/22, F16K 11/085, F16K 27/06

(54) **HAHN**
TAP
ROBINET

(30) Priorität: 30.10.2002 DE 10251644
(43) Veröffentlichungstag der Anmeldung: 27.07.2005
(73) Patentinhaber: Telegärtner Kuststofftechnik GmbH, 71144 Steinenbronn (DE)
(72) Erfinder: BLEYER, Georg, 71144 Steinenbronn (DE); HEINZELMANN, Frank, 72250 Freudenstadt (DE)
(74) Vertreter: Karrais, Martin
(86) Internationale Anmeldenummer: PCT/EP2003/011974
(87) Internationale Veröffentlichungsnummer: WO 2004/039446

(56) Entgegenhaltungen:
- US-A- 3 012 752
- US-A- 3 185 179
- US-A- 3 750 704
- US-A- 4 073 314
- US-A- 4 197 876
- US-A- 5 043 126

## Beschreibung

Die Erfindung betrifft einen Hahn mit den Merkmalen des Oberbegriffes von Patentanspruch 1.

Derartige Hähne kommen insbesondere in der Medizin zum Einsatz, beispielsweise bei der Verabreichung von Infusionen. Entsprechend der Drehstellung des Kükens relativ zum Gehäuse kann ein Medium durch Strömungskanäle des Gehäuses hindurchgeführt oder abgesperrt werden, es kann auch vorgesehen sein, daß das Gehäuse mehrere Strömungskanäle aufweist, so daß in Abhängigkeit von der Drehstellung des Kükens das Medium von einem Kanal in einen anderen Kanal umgelenkt werden kann.

In der DE 37 18 815 A1 wird vorgeschlagen, zur Herstellung des Hahnes das Küken in das in einem vorhergehenden Verfahrensschritt hergestellte Gehäuse einzuspritzen, wobei das Küken das Gehäuse oberseitig und unterseitig übergreift und dadurch unlösbar mit dem Gehäuse verbunden ist. Zur Erzielung einer zuverlässigen Abdichtung des durch den Hahn durchströmenden Mediums ist allerdings eine enge Passung zwischen Gehäuse und Küken erforderlich. Die hierzu erforderliche Maßgenauigkeit kann beim Spritzgießen des Kükens nicht in allen Fällen gewährleistet werden. Beim Einspritzen des Kükens in das Gehäuse besteht vor allem die Gefahr von Unrundheiten, die die Dichtheit des Hahnes beeinträchtigen.

Zur Vermeidung derartiger Undichtigkeiten wird in der DE 197 28 234 C2 eine separate Herstellung von Gehäuse und Küken vorgeschlagen. Um die erforderliche Dichtheit zu erzielen, umfaßt der aus dieser Druckschrift bekannte Hahn mindestens drei Dichtorgane, wobei zwei der Dichtorgane einen Schaft des Kükens in Umfangsrichtung vollständig umgeben und ein drittes Dichtorgan in axialer Richtung zwischen den beiden in Umfangsrichtung verlaufenden Dichtorgan angeordnet ist und mindestens eine Öffnung des Kükens ringartig umschließt. Beim Drehen des Kükens wird vor allem das dritte Dichtorgan erheblichen Scherkräften unterworfen, die die Dichtheit des Hahnes beeinträchtigen können. Die Festlegung des Kükens am Gehäuse erfolgt bei dieser Ausgestaltung mittels Rastelemente, die am Schaft des Kükens sowie im Bereich einer Durchgangsöffnung des Gehäuses angeordnet sind. Wird das Küken in das Gehäuse eingesetzt, so hintergreifen die Rastelemente einander. Um sicherzustellen, daß das Küken auf einfache Weise in das Gehäuse eingesetzt werden kann, ist es erforderlich, daß sich die Rastelemente beim Einsetzen nicht allzu sehr behindern. Damit besteht allerdings die Gefahr, daß das Küken aus dem Gehäuse herausgepreßt wird, wenn das den Hahn durchströmende Medium mit einem höheren Druck beaufschlagt wird.

Aus der US-A-3 750 704 ist ein Hahn mit einem Küken bekannt, das eine durchgehende Aufnahme aufweist, in die ein Verbindungselement eingesetzt ist. Das Verbindungselement überdeckt das Küken und ist mit einer Bodenwand des Gehäuses verrastet. Der obere Endabschnitt des Verbindungselements ist in Form eines Griffes ausgebildet zum Verdrehen des Kükens.

Aufgabe der vorliegenden Erfindung ist es, einen Hahn der eingangs genannten Art derart weiterzubilden, daß er eine hohe Dichtheit aufweist und dennoch leicht drehbar ist, ohne daß die Gefahr besteht, daß das Küken aus dem Gehäuse herausgepreßt wird.

Diese Aufgabe wird bei einem Hahn der gattungsgemäßen Art erfindungsgemäß dadurch gelöst, daß das Verbindungselement in die Aufnahme eingespritzt ist und mit einem oberen Endabschnitt den Griff und mit einem unteren Endabschnitt eine dem Griff abgewandte Unterseite des Gehäuses hintergreift. Eine derartige Ausgestaltung hat den Vorteil, daß das Küken und das Gehäuse in separaten Spitzgußformen hergestellt werden können, wobei jeweils eine sehr hohe Maßgenauigkeit erzielt werden kann. In einem weiteren Fertigungsschritt wird dann das Küken in das Gehäuse eingesetzt und in die Aufnahme des Kükens wird Kunststoffmaterial gespritzt, das ein das Küken unlösbar mit dem Gehäuse verbindendes Verbindungselement ausbildet. Die Aufnahme des Kükens kann einen unrunden Querschnitt aufweisen, so daß das die Aufnahme vorzugsweise vollständig ausfüllende Verbindungselement drehfest mit dem Küken verbunden ist. Die separate Formung des Kükens und des Gehäuses und die damit erzielbare hohe Maßgenauigkeit stellt sicher, daß der Hahn eine hohe Dichtheit aufweisen kann, wobei das Küken leicht drehbar ist. Um zu verhindern, daß insbesondere bei der Beaufschlagung des den Hahn durchströmenden Mediums mit einem hohen Druck das Küken aus dem Gehäuse herausgepreßt wird, kommt das Verbindungselement zum Einsatz. Das Verbindungselement ermöglicht nicht nur die Herstellung einer unlösbaren Verbindung zwischen Küken und Gehäuse, sondern es stellt außerdem sicher, daß der Griff des Hahnes unlösbar mit dem Küken verbunden ist. Der erfindungsgemäße Hahn hat somit auch den Vorteil, daß er kostengünstig herstellbar ist.

Zur Erzielung einer unlösbaren Verbindung zwischen Küken und Gehäuse ist bei einer bevorzugten Ausführungsform der Erfindung vorgesehen, daß die das Verbindungselement aufnehmende Aufnahme durch das Küken hindurchgeführt ist und daß das Verbindungselement die Aufnahme durchgreift und mit über die Aufnahme überstehenden oberen und unteren Endabschnitten das Küken und das Gehäuse hintergreift. Das Verbindungselement weist somit im wesentlichen eine Doppel-T-Form auf mit oberen und unteren Querabschnitten in Form der überstehenden Endabschnitte und mit einem das Küken durchgreifenden, parallel zur Drehachse des Kükens ausgerichteten Steg. Der Steg kann hierbei mehrere parallel zueinander ausgerichtete Stegabschnitte umfassen, die zwischen sich ein Bohrungssystem aufnehmen, das durch das Küken hindurchgeführt ist zur Aufnahme des den Hahn durchströmenden Mediums.

Bei einer kostengünstig herstellbaren Ausführungsform sind der Griff und das Küken einstückig miteinander verbunden. Es kann vorgesehen sein, daß der Griff eine Ausnehmung aufweist, in die die Aufnahme des Kükens einmündet, und daß das Verbindungselement sowohl in die Aufnahme des Kükens als auch in die Ausnehmung des Griffes eingespritzt ist.

Bei einer besonders bevorzugten Ausführungsform ist vorgesehen, daß das Verbindungselement mit einem oberen Endabschnitt ein Markierungselement ausbildet. Derartige Markierungselemente sind vorzugsweise an der Oberseite des Griffes angeordnet. Hierbei ist es günstig, wenn das Kunststoffmaterial des zumindest ein Markierungselement ausbildenden Verbindungselementes eingefärbt ist. Mittels des Markierungselementes kann die Drehstellung des Kükens relativ zum Gehäuse angezeigt werden. Wird der Hahn als Absperrelement ausgebildet, so kann mittels des Markierungselementes die geöffnete oder geschlossene Position des Hahnes angezeigt werden. Ist der Hahn als Mehrweghahn ausgebildet, so läßt sich mittels des Markierungselementes die jeweils durch die Drehstellung des Kükens vorgegebene Strömungsverbindung zwischen einzelnen Strömungskanälen des Gehäuses anzeigen.

Wie eingangs erwähnt, ermöglicht die separate Herstellung von Küken und Gehäuse eine hohe Maßgenauigkeit der beiden Teile, so daß zusammenwirkende Dichtflächen im Bereich des Gehäuses und des Kükens erzielt werden können, die dem Hahn eine hohe Dichtheit verleihen, ohne die leichte Drehbarkeit des Kükens zu beeinträchtigen. Um die Dichtheit zusätzlich zu verbessern kann vorgesehen sein, daß der Hahn zumindest ein Dichtungselement aus Kunststoff umfaßt, das das Küken in Umfangsrichtung umgibt und das in einen Randbereich einer das Küken aufnehmenden Öffnung des Gehäuses eingespritzt ist. Das zur Ausgestaltung des Dichtungselementes zum Einsatz kommende Kunststoffmaterial kann hierbei eine höhere Elastizität aufweisen als das Material des Gehäuses und/oder reibungsvermindernde Zusätze umfassen, so daß selbst bei Beaufschlagung des den Hahn durchströmenden Mediums mit einem hohen Druck eine Leckage des Hahnes zuverlässig verhindert wird und gleichzeitig sichergestellt ist, daß das Küken leicht drehbar ist.

Von besonderem Vorteil ist es, wenn der Hahn zwei im Abstand zueinander angeordnete Dichtungselemente umfaßt, wobei das Verbindungselement zumindest ein Dichtungselement hintergreift. Dies hat den Vorteil, daß das Verbindungselement auch der Festlegung des Dichtungselementes innerhalb der das Küken aufnehmenden Öffnung des Gehäuses dient. Von besonderem Vorteil ist es hierbei, wenn zwei in axialem Abstand zueinander angeordnete Dichtungselemente zum Einsatz kommen, die auf ihren einander abgewandten Seiten jeweils vom Verbindungselement hintergriffen werden.

Um die Handhabung des erfindungsgemäßen Hahnes zu vereinfachen ist es von Vorteil, wenn der Hahn zusammenwirkende Rastelemente aufweist, die in einer vorgegebenen Drehstellung des Kükens ineinander einrasten. So kann beispielsweise vorgesehen sein, daß die Rastelemente in einer Öffnungs- und/oder Schließstellung des Kükens einrasten, so daß der Benutzer bei der Bedienung des Hahnes das Erreichen einer gewünschten Drehstellung durch das Einrasten der Rastelemente erkennt.

Bevorzugt ist ein erstes Rastelement am Gehäuse und ein mit dem ersten Rastelement zusammenwirkendes zweites Rastelement am Verbindungselement angeordnet. So kann beispielsweise vorgesehen sein, daß das erste Rastelement als Rastaufnahme und das zweite Rastelement als Rastvorsprung ausgestaltet ist. Vorzugsweise ist der Rastvorsprung einstückig mit dem Verbindungselement verbunden.

Bei einer besonders vorteilhaften Ausführungsform der Erfindung weist das Küken zur Führung des durch den Hahn strömenden Mediums eine entlang eines Teilbereichs des Außenumfangs des Kükens verlaufende Nut auf. Es hat sich gezeigt, daß durch eine derartige Ausgestaltung ein besonders geringer Strömungswiderstand des Hahnes erzielt werden kann für das den Hahn durchströmende Medium.

Von Vorteil ist es hierbei, wenn die Nut in ihren Endbereichen abgerundet ist, denn dadurch kann im Übergangsbereich zwischen den Endbereichen der Nut und in das Gehäuse eingeformten Strömungskanälen ein quasi kontinuierlicher Übergang erzielt werden, so daß die Strömung des den Hahn durchströmenden Mediums im Übergangsbereich nicht merklich beeinträchtigt wird. Durch eine derartige Ausgestaltung kann insbesondere die Gefahr vermindert werden, daß sich im Übergangsbereich zwischen der Nut und den Strömungskanälen des Gehäuses Luftbläschen ansammeln, die vom strömenden Medium mitgeführt werden. Bei aus dem Stand der Technik bekannten Hähnen besteht häufig die Gefahr, daß derartige Luftbläschen sich im Übergangsbereich zwischen den Strömungskanälen des Gehäuses und dem Küken zu einer verhältnismäßig großen Luftblase zusammenlagern, die dann abgelöst und zu einem Patienten transportiert werden kann. Dies kann zu einer Gesundheitsgefährdung des Patienten führen. Wird jedoch sichergestellt, daß im Bereich des Kükens die Strömung des Mediums in Umfangsrichtung geführt wird, wobei die Endbereiche der das Medium aufnehmenden Nut abgerundet sind, so kann die Gefahr einer Ansammlung von Luftbläschen ganz erheblich vermindert werden.

Die nachfolgende Beschreibung bevorzugter Ausführungsformen der Erfindung dient im Zusammenhang mit der Zeichnung der näheren Erläuterung. Es zeigen:
- Figur 1:: eine schaubildliche Darstellung einer ersten Ausführungsform eines erfindungsgemäßen Hahnes schräg von oben;
- Figur 2:: eine schaubildliche Darstellung des Hahnes aus Figur 1 schräg von unten;
- Figur 3:: eine Draufsicht auf den Hahn gemäß Figur 1;
- Figur 4:: eine Schnittansicht längs der Linie 4-4 in Figur 3;
- Figur 5:: eine Schnittansicht längs der Linie 5-5 in Figur 3;
- Figur 6:: eine Schnittansicht längs der Linie 6-6 in Figur 4;
- Figur 7:: eine Schnittansicht entsprechend Figur 4 einer zweiten Ausführungsform eines erfindungsgemäßen Hahnes;
- Figur 8:: eine Schnittansicht entsprechend Figur 4 einer dritten Ausführungsform eines erfindungsgemäßen Hahnes und
- Figur 9:: eine Schnittansicht längs der Linie 9-9 in Figur 8.

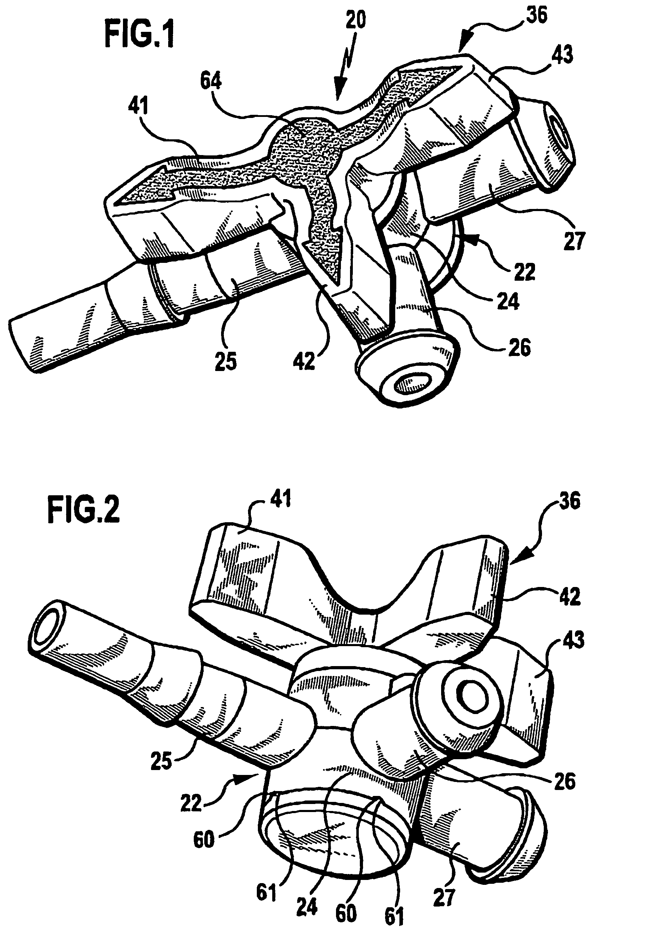

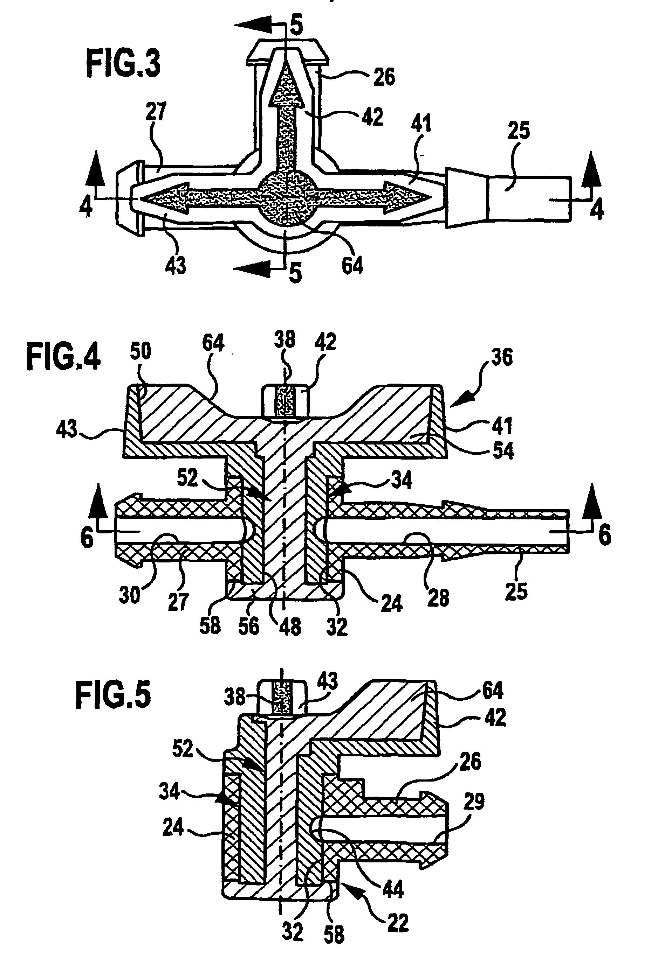

In den Figuren 1 bis 6 ist eine erste Ausführungsform eines erfindungsgemäßen Hahnes in Form eines insgesamt mit dem Bezugszeichen 20 belegten Drei-Wegehahns dargestellt. Dieser umfaßt ein Gehäuse 22 mit einem hohlzylinderförmigen Gehäusekörper 24, der einstückig mit drei Anschlußstutzen 25, 26, 27 verbunden ist. Letztere definieren jeweils einen Strömungskanal 28, 29 bzw. 30, der in eine den Gehäusekörper 24 durchgreifende Durchgangsöffnung 32 einmündet.

Der Drei-Wegehahn 20 umfaßt außerdem ein in die Durchgangsöffnung 32 eingesetztes Küken 34, das einstückig mit einem die Durchgangsöffnung 32 oberseitig überdeckenden Griff 36 verbunden ist. Das Küken 34 ist um eine Drehachse 38 drehbar, und der Griff 36 weist drei Flügel 41, 42, 43 auf, die parallel zu den Anschlußstutzen 25, 26 bzw. 27 ausgerichtet werden können.

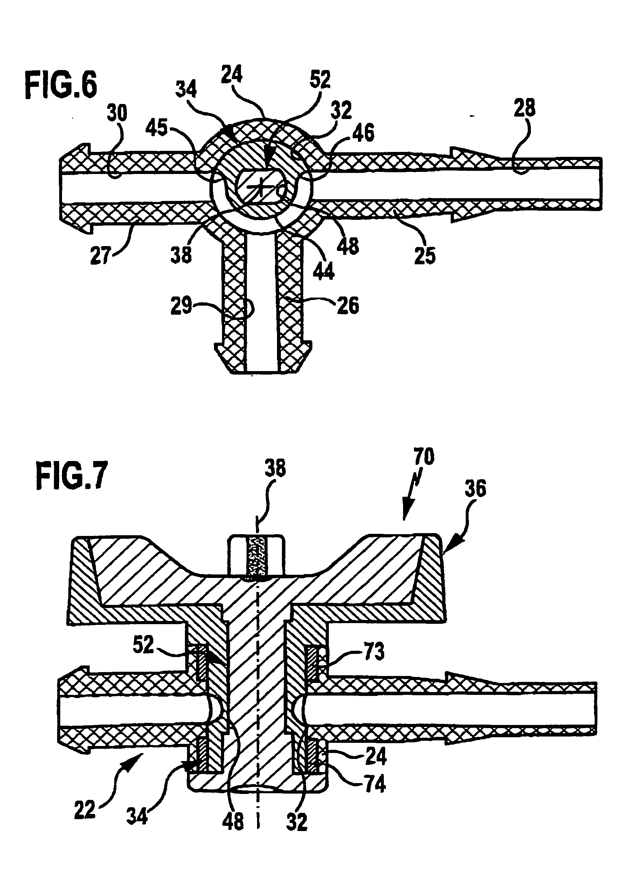

In Höhe der Strömungskanäle 28, 29, 30 weist das Küken 34 eine sich entlang eines Teilbereiches seines Außenumfangs erstreckende Nut 44 auf, deren Endbereiche 45, 46 abgerundet sind, so daß sich bei entsprechender Ausrichtung des Kükens 34 ein kontinuierlicher Übergang ergibt zwischen den Strömungskanälen 28, 29 und 30 und der Nut 44. Dies wird insbesondere aus Figur 6 deutlich.

Das Küken 34 weist eine koaxial zur Drehachse 38 ausgerichtete, durchgehende Aufnahme 48 auf, die auf der Oberseite des Griffes 36 in eine in den Griff 36 eingeformte Ausnehmung 50 übergeht und in die nach dem Einsetzen des Kükens 34 in die Durchgangsöffnung 32 des Gehäusekörpers 24 ein Kunststoffmaterial gespritzt ist, das ein Verbindungselement 52 ausbildet. Wie insbesondere aus Figur 6 deutlich wird, weist die Aufnahme 48 einen unrunden Querschnitt auf, so daß das Verbindungselement 52 drehfest mit dem Küken 34 verbunden ist.

Das Verbindungselement 52 durchgreift die Aufnahme 48 und hintergreift mit einem oberen Endabschnitt 54 den Griff 36 und mit einem unteren Endabschnitt 56 das dem Griff 36 abgewandte Ende des Kükens 34 sowie eine untere Stirnseite 58 des Gehäusekörpers 24. Der untere Endabschnitt 56 des Verbindungselementes 52 bildet eine Bodenwand des Gehäusekörpers 24 und weist der unteren Stirnseite 58 zugewandt drei Rastvorsprünge 60 auf, die in zugeordnete Rastaufnahmen 61 einrasten, die in die untere Stirnseite 58 eingeformt sind. Dies wird aus Figur 2 deutlich. Die Rastaufnahmen 61 sind in Umfangsrichtung in Höhe der Strömungskanäle 28, 29 und 30 angeordnet, so daß die Rastvorsprünge 60 in die zugeordneten Rastaufnahmen 61 einrasten, wenn zumindest ein Endbereich 45 oder 46 der Nut 44 des Kükens 34 mit einem Strömungskanal 28, 29 oder 30 fluchtet und somit eine Strömungsverbindung hergestellt ist zwischen einem Strömungskanal 28, 29 oder 30 und der Nut 44.

Die in den Griff 36 eingeformte Ausnehmung 50 erstreckt sich pfeilförmig entlang der Flügel 41, 42, 43, so daß das die Ausnehmung 50 ausfüllende Verbindungselement 52 im Bereich des Griffes 36 ein Markierungselement 64 ausbildet. Da das Kunststoffmaterial des Verbindungselementes 52 eingefärbt ist, ist das Markierungselement 64 auf der Oberseite des Griffes 36 für den Benutzer des Drei-Wegehahns 20 deutlich erkennbar.

Die Herstellung des Drei-Wegehahns 20 erfolgt dergestalt, daß in einem ersten Herstellungsschritt das Gehäuse 22 sowie das Küken 34 durch Spritzgießen hergestellt werden. In einem weiteren Herstellungsschritt wird dann das Küken 34 in die Durchgangsöffnung 32 des Gehäuses 22 eingesetzt und es wird dann in die Aufnahme 48 des Kükens 34 und in die Ausnehmung 50 des Griffes 36 ein Kunststoffmaterial gespritzt, das das Verbindungselement 52 ausbildet. Dieses hintergreift mit seinen oberen und unteren Endabschnitten 54 bzw. 56 den Griff 36 sowie das Küken 34, so daß letzteres unlösbar mit dem Gehäuse 22 verbunden ist.

Eine zweite Ausführungsform eines erfindungsgemäßen Drei-Wegehahnes ist in Figur 7 dargestellt und insgesamt mit dem Bezugszeichen 70 belegt. Dieser ist weitgehend identisch ausgebaut wie der voranstehend erläuterte Drei-Wegehahn 20. Für identische Bauteile wurden deshalb dieselben Bezugszeichen verwendet wie in den Figuren 1 bis 6, und zur Vermeidung Wiederholungen wird diesbezüglich vollinhaltlich auf die voranstehenden Erläuterungen verwiesen.

Der in Figur 7 dargestellte Drei-Wegehahn 70 unterscheidet sich vom Drei-Wegehahn 20 dadurch, daß die Durchgangsöffnung 32 des Gehäusekörpers 24 im Bereich ihrer axialen Endbereiche jeweils eine ringförmige Dichtungsaufnahme 71 und 72 aufweist, in die vor dem Einsetzen des Kükens 34 in das Gehäuse 22 jeweils ein Kunststoffmaterial eingespritzt ist, das ein oberes bzw. ein unteres Dichtungselement 73, 74 ausbildet. Mittels der oberhalb bzw. unterhalb der Nut 44 angeordneten, das Küken 34 in Umfangsrichtung umgebenden Dichtungselemente 73 und 74 wird selbst in den Fällen eine zuverlässige Dichtwirkung erzielt, in denen das den Drei-Wegehahn 70 durchströmende Medium mit einem hohen Druck beaufschlagt ist. Die Dichtungselemente 73 und 74 können hierbei reibungsvermindernde Zusätze enthalten, so daß sich das Küken 34 des Drei-Wegehahns 70 trotz des Einsatzes der Dichtungselemente 73 und 74 leicht drehen läßt.

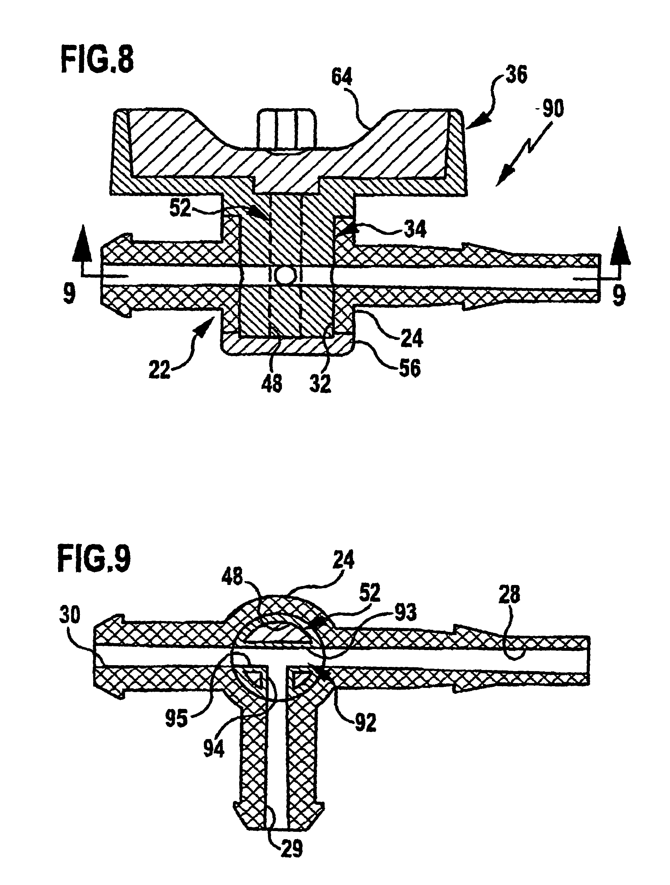

Eine weitere alternative Ausführungsform des erfindungsgemäßen Hahnes ist in den Figuren 8 und 9 in Form eines insgesamt mit dem Bezugszeichen 90 belegten Drei-Wegehahnes dargestellt. Dieser ist weitgehend identisch ausgestaltet wie der voranstehend unter Bezugnahme der Figuren 1 bis 6 erläuterte Drei-Wegehahn 20. Er unterscheidet sich von diesem lediglich dadurch, daß statt einer das Küken 34 entlang eines Teilbereiches seines Außenumfanges umgebenden Nut zur Führung des den Hahn 90 durchströmenden Mediums ein T-förmiges Bohrungssystem 92 das Küken 34 durchgreift mit drei Bohrungsabschnitten 93, 94, 95, die fluchtend zu den Strömungskanälen 28, 29 bzw. 30 ausgerichtet werden können.

Entsprechend der Ausgestaltung des Dref-Wegehahnes 20 wird auch das Küken 34 des Drei-Wegehahns 90 von dem in die Aufnahme 48 des Kükens 34 eingespritzten Verbindungselement 52 durchgriffen, das im Bereich des Griffs 36 mit einem oberen Endabschnitt das Markierungselement 64 ausbildet und den Griff 36 hintergreift und unterseitig eine Bodenwand des Gehäuses 22 ausbildet und mit seinem unteren Endabschnitt 56 sowohl das Küken 34 als auch die untere Stirnseite 58 des Gehäusekörpers 24 hintergreift. Mittels des Verbindungselementes 52 wird also auch beim Drei-Wegehahn 90 eine untrennbare Verbindung zwischen dem Küken 34 und dem Gehäuse 22 hergestellt.

## Patentansprüche

1. Hahn mit einem Gehäuse und einem darin drehbaren Küken, wobei Gehäuse (22) und Küken (34) aus Kunststoff gefertigt sind und das Küken (34) eine Aufnahme (48) aufweist, in der ein das Küken (34) unlösbar mit dem Gehäuse (22) verbindendes Verbindungselement (52) aus Kunststoff angeordnet ist, und wobei der Hahn (20; 70; 90) einen unverdrehbar mit dem Küken (34) verbundenen Griff (36) zum Verdrehen des Kükens (34) umfaßt, **dadurch gekennzeichnet, daß** das Verbindungselement (52) in die Aufnahme (48) eingespritzt ist und mit einem oberen Endabschnitt (54) den Griff (36) und mit einem unteren Endabschnitt (56) eine dem Griff (36) abgewandte Unterseite (58) des Gehäuses (22) hintergreift.

2. Hahn nach Anspruch 1, **dadurch gekennzeichnet, daß** die Aufnahme (48) durch das Küken (34) hindurchgeführt ist und daß das Verbindungselement (52) die Aufnahme (48) durchgreift und mit über die Aufnahme (48) überstehenden oberen und unteren Endabschnitten (54, 56) das Küken (34) und das Gehäuse (22) hintergreift.

3. Hahn nach Anspruch 1 oder 2, **dadurch gekennzeichnet, daß** der Griff (36) und das Küken (34) einstückig miteinander verbunden sind.

4. Hahn nach einem der voranstehenden Ansprüche, **dadurch gekennzeichnet, daß** das Verbindungselement (52) mit einem oberen Endabschnitt (54) ein Markierungselement (64) ausbildet.

5. Hahn nach einem der voranstehenden Ansprüche, **dadurch gekennzeichnet, daß** der Hahn (70; 80) zumindest ein Dichtungselement (73, 74) aus Kunststoff umfaßt, das das Küken (34) in Umfangsrichtung umgibt und das in einen Randbereich einer das Küken (34) aufnehmenden Öffnung (32) des Gehäuses (22) eingespritzt ist.

6. Hahn nach Anspruch 5, **dadurch gekennzeichnet, daß** der Hahn (70; 80) zwei im Abstand zueinander angeordnete Dichtungselemente (73, 74) umfaßt und daß das Verbindungselement (52) zumindest ein Dichtungselement (73, 74) hintergreift.

7. Hahn nach einem der voranstehenden Ansprüche, **dadurch gekennzeichnet, daß** der Hahn (20; 70; 80; 90) zusammenwirkende Rastelemente (60, 61) aufweist, die in einer vorgegebenen Drehstellung des Kükens (34) ineinander einrasten.

8. Hahn nach Anspruch 7, **dadurch gekennzeichnet, daß** ein erstes Rastelement (61) am Gehäuse (22) und ein mit dem ersten Rastelement (61) zusammenwirkendes zweites Rastelement (60) am Verbindungselement (52) angeordnet ist.

9. Hahn nach Anspruch 8, **dadurch gekennzeichnet, daß** das erste Rastelement als Rastaufnahme (61) und das zweite Rastelement als Rastvorsprung (60) ausgestaltet ist, wobei der Rastvorsprung (60) einstückig mit dem Verbindungselement (52) verbunden ist.

10. Hahn nach einem der voranstehenden Ansprüche, **dadurch gekennzeichnet, daß** das Küken (34) zur Führung des durch den Hahn strömenden Mediums eine entlang eines Teilbereichs seines Außenumfangs verlaufende Nut (44) aufweist.

11. Hahn nach Anspruch 10, **dadurch gekennzeichnet, daß** die Nut (44) in ihren Endbereichen (45, 46) abgerundet ist.

## Claims

1. A tap having a housing and a plug which can be rotated therein, the housing (22) and plug (34) being made of plastics material and the plug (34) having a receiving means (48) in which a connecting element (52) of plastics material, which connects the plug (34) non-detachably to the housing (22), is arranged, and the tap (20; 70; 90) comprising a handle (36), which is non-rotatably connected to the plug (34), for rotating the plug (34), **characterised in that** the connecting element (52) is injected into the receiving means (48) and reaches behind the handle (36) with an upper end portion (54) and behind an underside (58) of the housing (22), which is remote from the handle (36), with a lower end portion (56).

2. A tap according to Claim 1, **characterised in that** the receiving means (48) is guided through the plug (34) and **in that** the connecting element (52) reaches through the receiving means (48) and reaches behind the plug (34) and the housing (22) with upper and lower end portions (54, 56) projecting over the receiving means (48).

3. A tap according to Claim 1 or 2, **characterised in that** the handle (36) and the plug (34) are connected together in one piece.

4. A tap according to one of the preceding claims, **characterised in that** the connecting element (52) forms a marking element (64) with an upper end portion (54).

5. A tap according to one of the preceding claims, **characterised in that** the tap (70; 80) comprises at least one sealing element (73, 74) of plastics material, which surrounds the plug (34) in the circumferential direction and is injected into an edge region of an opening (32) in the housing (22), which receives the plug (34).

6. A tap according to Claim 5, **characterised in that** the tap (70; 80) comprises two sealing elements (73, 74) arranged at a spacing from one another and **in that** the connecting element (52) reaches behind at least one sealing element (73, 74).

7. A tap according to one of the preceding claims, **characterised in that** the tap (20; 70; 80; 90) has cooperating latching elements (60, 61) which latch inside one another in a predetermined rotary position of the plug (34).

8. A tap according to Claim 7, **characterised in that** a first latching element (61) is arranged on the housing (22) and a second latching element (60) cooperating with the first latching element (61) is arranged on the connecting element (52).

9. A tap according to Claim 8, **characterised in that** the first latching element is constructed as a latching receiving means (61) and the second latching element is constructed as a latching projection (60), the latching projection (60) being connected in one piece to the connecting element (52).

10. A tap according to one of the preceding claims, **characterised in that** the plug (34) has a groove (44) extending along a sub-region of its outer circumference for the purpose of conducting the medium flowing through the tap.

11. A tap according to Claim 10, **characterised in that** the groove (44) is rounded in its end regions (45, 46).

## Revendications

1. Robinet comportant un boîtier et une noix de robinet pouvant tourner à l'intérieur dudit boîtier, le boîtier (22) et la noix de robinet (34) étant réalisés en matière plastique et la noix de robinet (34) comportant un logement (48) dans lequel est agencé un élément de liaison (52) en matière plastique reliant de façon inamovible la noix de robinet (34) au boîtier (22), et le robinet (20 ; 70 ; 90) comportant une poignée (36) reliée à la noix de robinet (34), sans pouvoir tourner par rapport à celle-ci afin de pouvoir faire tourner la noix de robinet , **caractérisé en ce que** l'élément de liaison (52) est injecté dans le logement (48) et s'accroche par l'arrière, par une portion d'extrémité supérieure (54) avec la poignée (36), et par une portion d'extrémité inférieure (56) avec un côté inférieur (58), opposé à la poignée (36), du boîtier (22).

2. Robinet selon la revendication 1, **caractérisé en ce que** le logement (48) est ménagé au travers de la noix de robinet (34), et **en ce que** l'élément de liaison (52) est introduit dans le logement (48) et s'accroche par l'arrière, par des portions d'extrémité supérieure et inférieure (54, 56), saillant au-dessus du logement (48), avec la noix de robinet (34) et le boîtier (22).

3. Robinet selon la revendication 1 ou 2, **caractérisé en ce que** la poignée (36) et la noix de robinet (34) sont reliés l'un à autre d'une seule pièce.

4. Robinet selon l'une des revendications précédentes, **caractérisé en ce que** l'élément de liaison (52) comportant une portion d'extrémité supérieure (54) forme un élément de marquage (64).

5. Robinet selon l'une des revendications précédentes, **caractérisé en ce que** le robinet (70 ; 80) comporte au moins un élément d'étanchéité (73, 74) en matière plastique qui entoure la noix de robinet (34) dans la direction circonférentielle, et qui est injecté dans une région en bordure d'une ouverture (32), recevant la noix de robinet (34), du boîtier (22).

6. Robinet selon la revendication 5, **caractérisé en ce que** le robinet (70 ; 80) comporte deux éléments d'étanchéité (73, 74) agencés à distance l'un de l'autre, et **en ce que** l'élément de liaison (52) s'accroche par l'arrière avec au moins un élément d'étanchéité (73, 74).

7. Robinet selon l'une des revendications précédentes, **caractérisé en ce que** le robinet (20 ; 70 ; 80 ; 90) comporte des éléments d'encliquetage (60, 61) qui coopèrent l'un avec l'autre, et qui s'encliquètent l'un dans l'autre dans une position de rotation prescrite de la noix de robinet (34).

8. Robinet la revendication 7, **caractérisé en ce qu'**un premier élément d'encliquetage (61) est agencé au niveau du boîtier (22) et un deuxième élément d'encliquetage (60), coopérant avec le premier élément d'encliquetage (61), est agencé au niveau de l'élément de liaison (52).

9. Robinet selon la revendication 8, **caractérisé en ce que** le premier élément d'encliquetage est conformé en logement d'encliquetage (61), et le deuxième élément d'encliquetage est conformé en saillie d'encliquetage (60), la dite saillie d'encliquetage (60) étant reliée d'une seule pièce avec l'élément de liaison (52).

10. Robinet selon l'une des revendications précédentes, **caractérisé en ce que** la noix de robinet (34) comporte une gorge (44) s'étendant le long d'une région partielle de sa périphérie extérieure, afin de guider le fluide s'écoulant au travers du robinet.

11. Robinet selon la revendication 10, **caractérisé en ce que** la gorge (44) est arrondie dans ses régions d'extrémité (45, 46).
